# FASCICULE DE BREVET EUROPEEN

(11) **EP 3 096 065 B1**
(45) Date de publication et mention de la délivrance du brevet: **27.12.2017**
(21) Numéro de dépôt: 16170799.7
(22) Date de dépôt: 23.05.2016
(51) Int. Cl.: A61B 90/50, A61B 46/10, F16M 11/14, F16M 11/20, F16M 11/24, F16M 11/40, F16M 11/42, A61B 90/00

(54) **SYSTÈME VIDÉO COMPRENANT UNE ARMATURE ARTICULÉE**
VIDEOSYSTEM, DAS EINEN RAHMEN MIT GELENKVERBINDUNG UMFASST
VIDEO SYSTEM INCLUDING A HINGED FRAME

(30) Priorité: 21.05.2015 FR 1554593
(43) Date de publication de la demande: 23.11.2016
(73) Titulaire: Euromedis Groupe, 60290 Neuilly Sous Clermont (FR)
(72) Inventeur: LERICHE, Jean-Paul, 60600 BREUIL LE VERT (FR)
(74) Mandataire: Hirsch & Associés

(56) Documents cités:
- EP-A1- 1 239 805
- WO-A1-98/49944
- WO-A1-03/002011
- WO-A1-2015/042460
- WO-A2-2010/102197
- FR-A1- 3 006 418

## Description

La présente invention concerne un système vidéo comprenant une armature articulée, ladite armature comportant notamment un bras articulé et un dispositif articulé.

Le domaine technique de l'invention est celui de la prise de vues. Plus particulièrement, l'invention se rapporte à la visualisation et/ou à l'enregistrement d'images au cours d'actes médicaux.

Dans l'art antérieur, on connait notamment des systèmes vidéo fixés dans des blocs opératoires. De tels systèmes vidéo comportent une caméra et un écran d'affichage des images filmées par ladite caméra. Typiquement la caméra est disposée au bout d'un bras articulé fixé au plafond et l'écran d'affichage est fixé contre un mur. Le plus souvent, un opérateur se consacre à la commande de la caméra. Le document FR 3 006 418 A1 montre une armature articulée selon le préambule de la revendication 1.

Pour équiper un hôpital ou une clinique, il convient donc, à l'heure actuelle, de prévoir autant de systèmes vidéo que de blocs opératoires, ce qui pose un problème évident de coût.

En outre, les blocs opératoires sont généralement dotés de plusieurs appareils médicaux qui peuvent être volumineux et disposés en particulier à proximité de la table d'opération.

De ce fait, un tel bras articulé de système vidéo oblige le personnel médical à prendre de nombreuses précautions, notamment en adaptant la position de la table d'opérations et/ou des autres appareils médicaux en fonction de l'endroit où ledit système vidéo est fixé.

Il existe donc un réel besoin de résolution des problèmes techniques ici exposés.

A cette fin, la présente invention propose une armature articulée de système vidéo destiné à être disposé dans un bloc opératoire, ladite armature comprenant
- un socle comportant des moyens de déplacement pour déplacer ladite armature,
- un mât fixé audit socle et s'étendant suivant une direction principale verticale,
- un dispositif articulé solidaire dudit mât et dont l'extrémité opposée audit mât est mobile dans trois dimensions, ladite extrémité étant destinée à supporter un écran d'affichage, ledit dispositif présentant une première portée, et
- un bras articulé solidaire dudit mât et dont l'extrémité opposée audit mât est mobile dans trois dimensions, ladite extrémité étant destinée à supporter un appareil de prise de vues, ledit bras présentant une deuxième portée strictement supérieure à ladite première portée.

Grâce à ces dispositions, le confort pour la prise de vues d'opérations chirurgicales et leur visionnage est optimal.

Selon un mode de réalisation de cette armature articulée, le bras articulé comporte
- au moins deux éléments tubulaires, lesdits éléments tubulaires recevant des câbles électriques destinés à être reliés à l'appareil de prise de vues, lesdits éléments tubulaires étant adaptés pivoter autour d'axes verticaux et horizontaux, et
- une poignée et un support aménagés, via un dispositif d'orientation, à l'extrémité mobile de l'élément tubulaire opposé au mât de manière à orienter l'appareil de prise de vues, ledit élément tubulaire où sont aménagés ladite poignée et ledit support présentant une longueur strictement inférieure à la longueur de l'autre élément tubulaire dudit bras articulé.

Grâce à ces dispositions, la caméra peut pivoter à 360° sans rencontrer d'obstacle et l'asepsie est facilitée.

Selon un autre mode de réalisation de cette armature articulée, le dispositif d'orientation comporte une rotule autour de laquelle pivote un cylindre présentant au moins un alésage taraudé recevant une vis dont la tête est une molette de serrage de ladite rotule.

Grâce à ces dispositions, la maniabilité de la caméra est améliorée.

Selon encore un mode de réalisation de cette armature articulée, le dispositif d'orientation comporte
- plusieurs pièces, appelées olives, présentant chacune une surface convexe et une surface concave complémentaires entre elles,
- un insert dont une partie est filetée et aménagé au sein de l'élément tubulaire, et
- une molette de serrage comportant un taraudage recevant la partie filetée dudit insert de manière à régler la flexibilité dudit élément tubulaire.

Selon encore un autre mode de réalisation de cette armature articulée, au moins une partie du bras est réalisée dans un matériau amagnétique.

Grâce à ces dispositions, les émissions de champs électromagnétiques du bras articulé sont minimisées et ainsi les risques de dysfonctionnement des autres appareils électroniques du bloc opératoire dans lequel est installée l'armature sont réduits.

Selon des caractéristiques particulières, le dispositif articulé comporte
- un support aménagé à l'extrémité mobile dudit dispositif, ledit support étant destiné à supporter l'écran d'affichage,
- plusieurs articulations munies de molettes de blocage,
- une tige s'étendant de façon sensiblement verticale et présentant une butée à chacune de ses extrémités,
- un tube disposé autour de ladite tige et présentant un alésage latéral taraudé, et
- une vis disposée au sein dudit alésage et dont la tête est une molette de serrage de ladite tige.

Grâce à ces dispositions, le réglage de l'orientation de l'écran d'affichage est précis, simple et rapide.

Selon des caractéristiques particulières, le socle lesté et en ce que les moyens de déplacement sont des roues montées sur ledit socle et dont la rotation est blocable.

Grâce à ces dispositions, la mobilité et la stabilité de l'armature articulée sont optimales.

Selon des caractéristiques particulières, cette armature articulée comporte en outre un accumulateur électrique destiné à alimenter en électricité l'appareil de prise de vues et/ou l'écran d'affichage.

Grâce à ces dispositions, l'armature articulée est énergétiquement autonome.

L'invention a également pour objet un système vidéo destiné à être disposé dans un bloc opératoire, ledit système vidéo comprenant
- une armature articulée selon l'une des revendications précédentes,
- l'appareil de prise de vues supporté par le bras articulé, et
- l'écran d'affichage fixé sur l'appui articulé.
Les avantages liés au système vidéo, étant similaires à ceux de l'armature articulée selon l'invention, ne sont pas rappelés ici.

Selon des caractéristiques particulières, ce système vidéo comporte en outre
- des moyens de communication, et
- un dispositif d'enregistrement des vues prises par l'appareil de prise de vues. Les inventeurs ont déterminé que ces dispositions sont optimales.

D'autres caractéristiques et avantages de l'invention apparaîtront à la lecture de la description qui suit d'un mode de réalisation préféré de l'invention, donnée à titre d'exemple et en référence au dessin annexé, qui montre :
- figure 1, une vue d'ensemble en perspective oblique d'un exemple de système vidéo selon l'invention ;
- figure 2, une vue éclatée en perspective oblique d'un premier exemple d'une partie d'un bras articulé selon l'invention ;
- figure 3, une vue éclatée en perspective oblique d'un deuxième exemple de la même partie du bras articulé selon l'invention ;
- figure 4, une vue éclatée en perspective oblique d'un exemple de dispositif articulé selon l'invention.

Dans la suite de la description, les éléments identiques ou de fonction identique portent le même signe de référence. À fin de concision de la présente description, les éléments identiques aux différents exemples ne sont pas décrits en regard de chacun de ces exemples. En d'autres termes, seules les différences entre les différents exemples sont décrites de manière détaillée, les éléments communs étant décrits en regard d'un seul exemple.

Par ailleurs, les valeurs numériques mentionnées ci-après, quoique non limitatives, se sont révélées aux essais comme fournissant les résultats les plus avantageux.

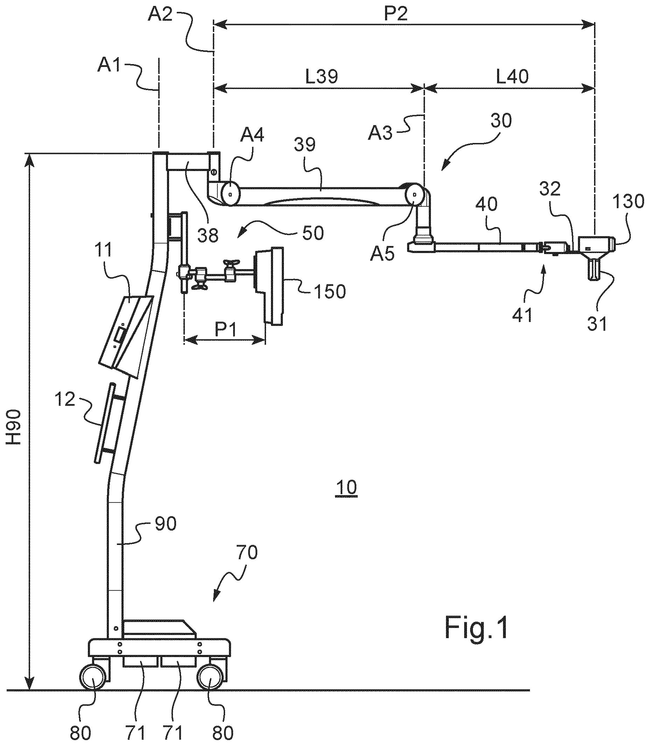

La figure 1 représente un mode de réalisation d'un système vidéo 10 comprenant une armature articulée 20 sur laquelle sont disposés un appareil 130 de prise de vues et un écran 150 d'affichage. Typiquement, le système vidéo 10 est destiné à être installé dans un bloc opératoire.

L'armature articulée 20 comprend un socle 70 sur lequel est fixé un mât 90 s'étendant suivant une direction principale verticale. Le socle 70 comporte des moyens de déplacement, ici des roues 80, pour déplacer le système vidéo 10.

Le système vidéo 10 comporte en outre un boîtier 11 et une poignée 12, réalisée de préférence en inox, fixés au mât 90. Le boîtier 11 comporte notamment des moyens de communication, un convertisseur de signaux vidéo et un dispositif d'enregistrement des vues prises par la caméra 130. Les moyens de communication comprennent par exemple une entrée/sortie pour l'acquisition/exportation de vidéos de haute définition. La poignée 12 facilite le déplacement du système vidéo 10.

De préférence, le diamètre des roues 80 est maximisé pour faciliter le déplacement du système vidéo 10 quel que soit la nature et la régularité du sol. Par ailleurs, la rotation des roues 80 est blocable pour stabiliser le système vidéo 10 lorsqu'il est correctement positionné pour fonctionner.

Dans l'exemple, le mât 90 présente une hauteur H90 d'environ deux mètres et le socle 70 est lesté au moyen de lests 71. En outre, le socle 70 comporte un accumulateur électrique destiné à alimenter en électricité notamment la caméra 130, l'écran 150 d'affichage, les moyens de communication et le dispositif d'enregistrement. De préférence, l'alimentation en électricité est sécurisée au moyen de deux fusibles bipolaires.

L'armature articulée 20 comprend en outre un dispositif articulé 50 solidaire du mât 90 et dont l'extrémité opposée audit mât est mobile dans trois dimensions.

L'extrémité de l'armature articulée 20 opposée au mât 90 supporte un écran 150 d'affichage. Dans l'exemple, l'écran 150 d'affichage présente une diagonale d'une longueur de quinze pouces.

Le dispositif articulé 50 présente une portée P1 de l'ordre de trente-cinq centimètres.

L'armature articulée 20 comprend en outre un bras articulé 30 solidaire du mât 90 et dont l'extrémité opposée audit mât est, comme l'extrémité du dispositif articulé 50, mobile dans trois dimensions.

De préférence, au moins une partie du bras articulé 30 est réalisée dans un matériau amagnétique.

L'extrémité du bras articulé 30 opposée au mât 90 supporte un appareil 130 de prise de vues, ici une caméra.

Le bras articulé 30 présente une portée P2, ici comprise entre un et deux mètres, strictement supérieure à la portée du dispositif articulé 50.

Le bras articulé 30 comporte une poignée 31 et un support 32 aménagés, via un dispositif d'orientation 41, à l'extrémité mobile dudit bras de manière à orienter la caméra 130. De préférence, la poignée 31 est recouverte d'une protection stérile ou stérilisable à usage unique.

Le bras articulé 30 comporte en outre plusieurs, ici trois, éléments tubulaires 38, 39 et 40 recevant des câbles électriques reliés à la caméra 130. Les câbles permettent d'alimenter en électricité et commander la caméra 130. De préférence, les câbles de commande de la caméra 130 sont reliés à une pédale disposée au pied du système vidéo 10 de manière à être actionnée par un opérateur pour contrôler le zoom de l'appareil de prise de vues 130, le démarrage et l'arrêt des prises de vues ainsi que leur enregistrement, leur diffusion et leur affichage.

L'élément tubulaire 38 est fixé au mât 90, tandis que les éléments tubulaires 39 et 40 pivotent autour d'axes verticaux A2 et A3, respectivement et d'axes horizontaux A4 et A5, respectivement. L'élément tubulaire 40 où sont aménagés la poignée 31 et le support 32 présente une longueur L40, ici de l'ordre d'une soixantaine de centimètres, strictement inférieure à la longueur L39, ici de l'ordre d'un mètre, de l'autre élément tubulaire 39 du bras articulé 30.

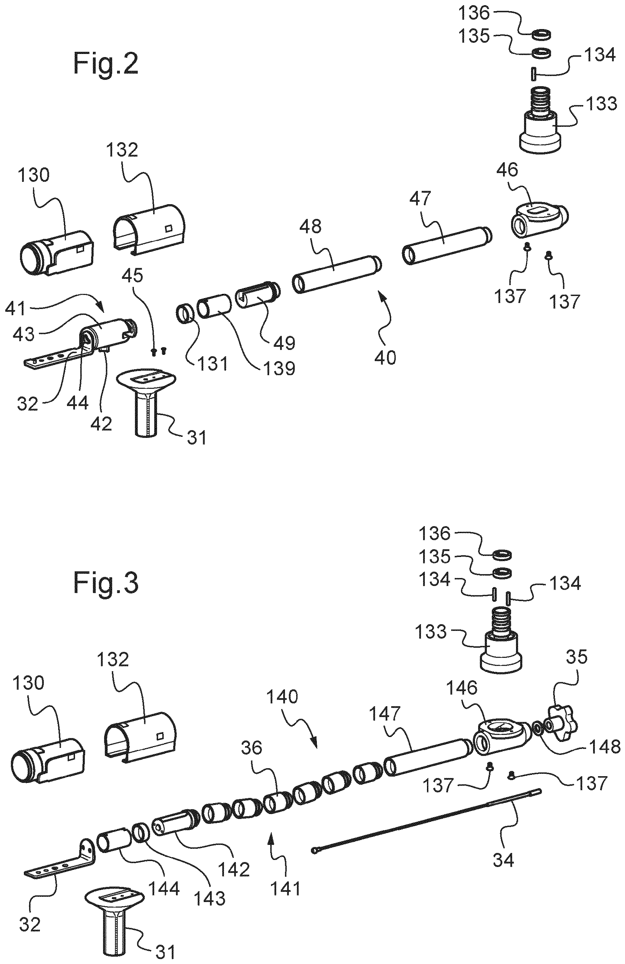

La figure 2 représente une vue éclatée en perspective oblique d'un premier exemple d'une partie du bras articulé 30 selon l'invention.

Le dispositif d'orientation 41 du bras articulé 30 comporte une rotule autour de laquelle pivote un cylindre 43 présentant au moins un alésage taraudé, ici deux, recevant une vis dont la tête est une molette de serrage 42 et 44 de ladite rotule.

De préférence, la rotule est une sphère fixée à une tige et le cylindre 43 comporte une encoche adaptée à recevoir ladite tige lorsqu'un opérateur incline la caméra vers le sol. En outre, la caméra 130 est couverte par un capot 132 de protection, ici réalisé en inox et muni d'ouïes d'aération.

Typiquement, la poignée 31 et le capot 132 de protection sont fixés au support 32 au moyen d'au moins une vis 45.

Dans l'exemple, le bras articulé 30 comporte plusieurs tubes 46-48 et un tube de jonction 49 muni d'un anneau passe-câbles 139 et une rondelle passe-câbles 131 emboîtés les uns dans les autres pour former l'élément tubulaire 40 du bras articulé 30.

Le tube 46 le plus éloigné de la caméra 130 présente une ouverture supérieure pour recevoir les câbles s'étendant le long de l'axe A3 et au sein d'un élément tubulaire vertical 133 de connexion avec l'élément tubulaire 39 le plus éloigné de la caméra 130. L'élément tubulaire vertical 133 est doté d'au moins une goupille 134, ici réalisées en inox, et de bagues tournantes fendues 135 et 136, ici réalisées en plastique. L'élément tubulaire vertical 133 est fixé au tube 46 le plus éloigné de la caméra 130 au moyen d'au moins une vis 137.

La figure 3 représente une vue éclatée en perspective oblique d'un deuxième exemple de la même partie d'un bras articulé 130 selon l'invention.

Le bras articulé 30 comporte ici un dispositif d'orientation 141 et, de part et d'autre dudit dispositif d'orientation, un élément tubulaire 140. L'élément tubulaire 140 et le dispositif d'orientation 141 reçoivent les câbles électriques reliés à la caméra 130.

Le dispositif d'orientation 141 comporte plusieurs, ici six, pièces 36, appelées olives, présentant chacune une surface convexe et une surface concave complémentaires entre elles.

Le dispositif d'orientation 141 comporte en outre
- un insert 34 dont une partie est filetée et aménagé au sein de l'élément tubulaire 140,
- une molette 35 de serrage comportant un taraudage recevant la partie filetée de l'insert 34 de manière à régler la flexibilité de l'élément tubulaire 140, et
- un roulement à billes 148 disposé entre ledit insert 34 et ladite molette 35.

L'élément tubulaire 140 comporte, entre les olives 36 et le support 32, un tube de jonction 142 présentant une surface convexe orientée vers lesdites olives. Le tube 142 est muni d'un anneau passe-câbles 143 et d'une rondelle passe-câbles 144.

L'élément tubulaire 140 comporte en outre, du côté des olives 36 opposé au support 32, un tube 147 et un tube 146 présentant une ouverture supérieure pour recevoir les câbles s'étendant le long de l'axe A3 et au sein de l'élément tubulaire vertical 133 de connexion avec l'élément tubulaire 39 le plus éloigné de la caméra 130.

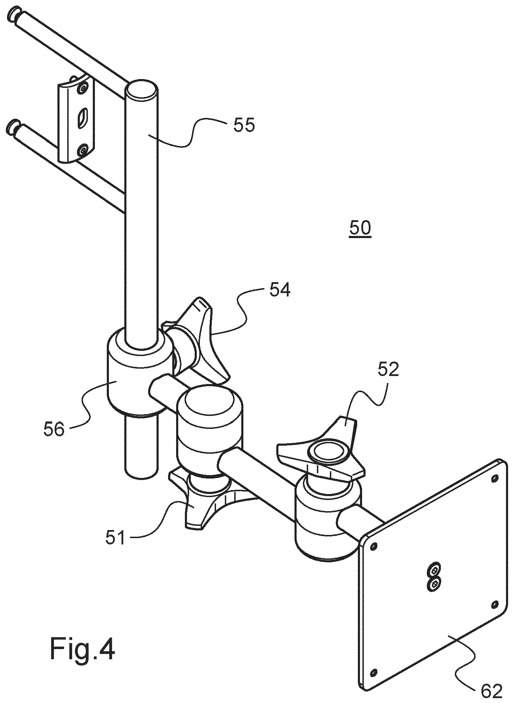

La figure 4 représente une vue éclatée en perspective oblique d'un exemple du dispositif articulé 50 selon l'invention.

Le dispositif articulé 50 comporte un support 62 aménagé à l'extrémité mobile dudit dispositif, ledit support étant destiné à supporter l'écran 150 d'affichage.

Le dispositif articulé 50 comporte en outre plusieurs, ici deux, articulations munies de molettes de blocage 51 et 52.

Le dispositif articulé 50 comporte enfin
- une tige 55 s'étendant de façon sensiblement verticale et présentant une butée à chacune de ses extrémité,
- un tube 56 disposé autour de ladite tige et présentant un alésage latéral taraudé, et
- une vis disposée au sein dudit alésage et dont la tête est une molette de serrage 54 de ladite tige.

Dans l'exemple, la tige 55 est fixée par vissage au mât 90 au moyen de deux barres parallèles entre elles et séparées par un entretoise.

Bien entendu, la présente invention n'est pas limitée aux exemples décrits et représentés, mais elle est susceptible de nombreuses variantes accessibles à l'homme de l'art.

## Revendications

1. Armature articulée (20) de système vidéo (10) destiné à être disposé dans un bloc opératoire, ladite armature comprenant
- un socle (70) comportant des moyens de déplacement pour déplacer ladite armature,
- un mât (90) fixé audit socle et s'étendant suivant une direction principale verticale,
- un dispositif articulé (50) solidaire dudit mât et dont l'extrémité opposée audit mât est mobile dans trois dimensions, ladite extrémité étant destinée à supporter un écran (150) d'affichage, ledit dispositif présentant une première portée (P1), et
- un bras articulé (30) solidaire dudit mât et dont l'extrémité opposée audit mât est mobile dans trois dimensions, ladite extrémité étant destinée à supporter un appareil (130) de prise de vues, ledit bras présentant une deuxième portée (P2) strictement supérieure à ladite première portée et comportant:
au moins deux éléments tubulaires (39 ; 40 ; 140), lesdits éléments tubulaires recevant des câbles électriques destinés à être reliés à l'appareil (130) de prise de vues, lesdits éléments tubulaires étant adaptés pour pivoter autour d'axes verticaux (A2 ; A3) et horizontaux (A4 ; A5), et
**caractérisé en ce que** le bras articulé comporte en plus une poignée (31) et un support (32) aménagés, via un dispositif d'orientation (41 ; 141), à l'extrémité mobile de l'élément tubulaire (40 ; 140) opposé au mât (90) de manière à orienter l'appareil (130) de prise de vues, ledit élément tubulaire où sont aménagés ladite poignée et ledit support présentant une longueur (L40) strictement inférieure à la longueur (L39) de l'autre élément tubulaire (39) dudit bras articulé, en sorte de permettre à un utilisateur de faire pivoter la caméra à 360° sans rencontrer d'obstacle tout en maintenant de bonnes conditions d'asepsie.

2. Armature articulée (20) selon la revendication 1, **caractérisée en ce que** le dispositif d'orientation (41) comporte une rotule autour de laquelle pivote un cylindre (43) présentant au moins un alésage taraudé recevant une vis dont la tête est une molette de serrage (42) de ladite rotule.

3. Armature articulée (20) selon la revendication 2, **caractérisée en ce que** la rotule est une sphère fixée à une tige, et le cylindre (43) comporte une encoche apte à recevoir ladite tige lorsque l'appareil (130) de prise de vues est incliné vers le sol.

4. Armature articulée (20) selon la revendication 1, **caractérisée en ce que** le dispositif d'orientation (141) comporte
- plusieurs pièces (36), appelées olives, présentant chacune une surface convexe et une surface concave complémentaires entre elles,
- un insert (34) dont une partie est filetée et aménagé au sein de l'élément tubulaire (40 ; 140), et
- une molette (35) de serrage comportant un taraudage recevant la partie filetée dudit insert de manière à régler la flexibilité dudit élément tubulaire.

5. Armature articulée (20) selon l'une des revendications 1 à 3, **caractérisée en ce qu'**au moins une partie du bras articulé (30) est réalisée dans un matériau amagnétique.

6. Armature articulée (20) selon l'une des revendications 1 à 4, **caractérisée en ce que** le dispositif articulé (50) comporte
- un support (62) aménagé à l'extrémité mobile dudit dispositif, ledit support étant destiné à supporter l'écran (150) d'affichage,
- plusieurs articulations munies de molettes de blocage (51 ; 52),
- une tige (55) s'étendant de façon sensiblement verticale et présentant une butée à chacune de ses extrémités,
- un tube (56) disposé autour de ladite tige et présentant un alésage latéral taraudé, et
- une vis disposée au sein dudit alésage et dont la tête est une molette de serrage (54) de ladite tige.

7. Armature articulée (20) selon l'une des revendications 1 à 5, **caractérisée en ce que** le socle (70) lesté et **en ce que** les moyens de déplacement sont des roues (80) montées sur ledit socle et dont la rotation est blocable.

8. Armature articulée (20) selon l'une des revendications 1 à 6, **caractérisée en ce qu'**elle comporte en outre un accumulateur électrique destiné à alimenter en électricité l'appareil (130) de prise de vues et/ou l'écran (150) d'affichage.

9. Système vidéo (10) destiné à être disposé dans un bloc opératoire, ledit système vidéo comprenant en outre
- une armature articulée (20) selon l'une des revendications précédentes,
- l'appareil (130) de prise de vues supporté par le bras articulé (30), et
- l'écran (150) d'affichage fixé sur l'appui articulé (50).

10. Système vidéo (10) selon la revendication 8, **caractérisé en ce qu'**il comporte
- des moyens de communication, et
- un dispositif d'enregistrement des vues prises par l'appareil (130) de prise de vues.

## Patentansprüche

1. Artikulierte Armatur (20) für Videosysteme (10), das für eine Anwendung in einem Operationssaal vorgesehen ist, wobei die Armatur Folgendes umfasst:
- einen Sockel (70) der Bewegungsmittel zum Bewegen der Armatur umfasst,
- einen Mast (90) der an dem Sockel befestigt ist und sich gemäß einer senkrechten Hauptrichtung erstreckt,
- eine Gelenkvorrichtung (50) die einstückig mit dem Mast ausgebildet ist und deren, dem Mast entgegengesetztes Ende dreidimensional bewegbar ist, wobei das Ende zum Abstützen eines Bildschirms (150) vorgesehen ist, und wobei die Vorrichtung eine erste Reichweite (P1) aufweist, und
- einen Gelenkarm (30) der einstückig mit dem Mast ausgebildet ist, und dessen, dem Mast entgegengesetztes Ende dreidimensional bewegbar ist, wobei das Ende zum Abstützen eines Bildaufnahmegeräts (130) vorgesehen ist, und wobei der Arm eine zweite Reichweite (P2) aufweist, die strikt grösser als die erste Reichweite ist, und die Folgendes umfasst:
- mindestens zwei Rohrelemente (39; 40; 140), wobei die Rohrelemente elektrische Kabel aufnehmen, die dazu bestimmt sind an das Bildaufnahmegerät (130) angeschlossen zu werden, und wobei die Rohrelemente drehbar um die senkrechten Achsen (A2; A3) und die vertikalen Achsen (A4; A5) angepasst sind, und
**dadurch gekennzeichnet dass** der Gelenkarm ferner Folgendes umfasst:
- einen Griff (31) und eine Abstützung (32), die durch eine Ausrichtungsvorrichtung (41; 141), an dem, dem Mast (90) entgegengesetzten beweglichen Ende des Rohrelements (40; 140) angeordnet sind, um das Bildaufnahmegerät (130) zu lenken, wobei das Rohrelement, an welchem der Griff und die Abstützung angeordnet sind, eine strikt kleinere Länge (L40) als die Länge (L39) des anderen Rohrelements (39) des Arms aufweist, um es einem Benutzer zu ermöglichen, die Kamera, ungehindert, unter Wahrung guter aseptischer Bedingungen, um 360° zu schwenken.

2. Artikulierte Armatur (20) nach Anspruch 1, **dadurch gekennzeichnet, dass** die Ausrichtungsvorrichtung (41) ein Kugelgelenk umfasst, um welches ein Zylinder (43) schwenkt, der mindestens eine Gewindebohrung aufweist, die eine Schraube aufnimmt, dessen Kopf ein Spannrad (42) des Kugelgelenks ist.

3. Artikulierte Armatur (20) nach Anspruch 2, **dadurch gekennzeichnet, dass** das Kugelgelenk eine, an einer Stange befestigte Kugel ist, und der Zylinder (43) eine Einkerbung umfasst, welche die Stange aufnehmen kann wenn das Bildaufnahmegerät (130) zum Boden geneigt ist.

4. Artikulierte Armatur (20) nach Anspruch 1, **dadurch gekennzeichnet, dass** die Ausrichtungsvorrichtung (141) Folgendes umfasst
- mehrere Teile (36), Oliven genannt, wobei jede davon eine konvexe Oberfläche und eine konkave Oberfläche aufweist, die miteinander komplementär sind,
- einen Einsatz (34), von dem ein Abschnitt mit einem Gewinde versehen und innerhalb des Rohrelements (40; 140) angebracht ist, und
- ein Spannrad (35) das ein Gewinde umfasst, das den Gewindeabschnitt des Einsatzes aufnimmt, um die Flexibilität des Rohrelemente zu regeln.

5. Artikulierte Armatur (20) nach einem der Ansprüche 1 bis 3, **dadurch gekennzeichnet, dass** mindestens ein Teil des Gelenkarms (30) aus einem nichtmagnetischem Material hergestellt ist.

6. Artikulierte Armatur (20) nach einem der Ansprüche 1 bis 4, **dadurch gekennzeichnet, dass** die Gelenkvorrichtung (50) Folgendes umfasst
- eine Abstützung (62), die an dem beweglichen Ende der Gelenkvorrichtung angeordnet ist, wobei die Abstützung dazu bestimmt ist, den Bildschirms (150) zu tragen.
- mehrere, mit Verriegelungsrädern (51; 52) versehene Gelenke,
- eine Stange (55), die sich im Wesentlichen senkrecht erstreckt und einen Anschlag an jedem seiner Enden aufweist,
- ein Rohr (56), das um die Stange herum ausgerichtet ist und eine seitliche Gewindebohrung aufweist, und
- eine Schraube, die innerhalb der Bohrung angebracht ist und dessen Kopf ein Spannrad (54) der Stange ist.

7. Artikulierte Armatur (20)) nach einem der Ansprüche 1 bis 5, **dadurch gekennzeichnet, dass** der Sockel (70) gewichtet ist und dass die Bewegungsmittel an dem Sockel befestigte Räder (80) sind, deren Drehung blockierbar ist.

8. Artikulierte Armatur (20) nach einem der Ansprüche 1 bis 6, **dadurch gekennzeichnet, dass** sie ferner einem elektrischen Akkumulator umfasst, der dazu vorgesehen ist, das Bildaufnahmegerät (130) und/oder den Bildschirm (150) mit Strom zu versorgen.

9. Videosystem (10), das dazu vorgesehen ist, in einem Operationsaal angeordnet zu werden, wobei das Videosystem ferner Folgendes umfasst
- eine artikulierte Armatur (20), nach einem der vorhergehenden Ansprüche,
- das Bildaufnahmegerät (130), das vom Gelenkarm (30) getragen wird, und
- den Bildschirm (150) der auf einer gelenkig angeordneten Auflage (50) angebracht ist.

10. Videosystem (10), nach Anspruch 8, **dadurch gekennzeichnet, dass** es Folgendes umfasst
- Kommunikationsmittel, und
- eine Vorrichtung für die Aufnahme der, von dem Bildaufnahmegerät (130) aufgenommenen Bildaufnahmen.

## Claims

1. A hinged frame (20) of a video system (10) intended to be disposed in an operating room, said frame comprising:
- a base (70) including displacement means for displacing said frame,
- a mast (90) attached to said base and extending along a vertical main direction,
- a hinged device (50) integral with said mast and of which the end opposite said mast is movable in three dimensions, said end being intended to support a display screen (150), said device having a first span (P1), and
- a hinged arm (30) integral with said mast and of which the end opposite said mast is movable in three dimensions, said end being intended to support and image forming device (130), said arm having a second span (P2) strictly greater than said first span and including at least two tubular elements (39; 40; 140), said tubular elements receiving electrical cables intended to be connected to the image forming device (130), said tubular elements being suitable for pivoting around vertical (A2; A3) and horizontal (A4; A5) axes, and
**characterized in that** the hinged arm also includes
a handle (31) and a support (32) arranged, via an orientation device (41; 141), at the movable end of the tubular element (40; 140) opposite the mast (90) so as to orient the image forming device (130), said tubular element where said handle and said support are arranged having a length (L40) strictly smaller than the length (L39) of the other tubular element (39) of said hinged arm,
so as to allow a user to pivot the camera 360° without encountering an obstacle while maintaining good sterile conditions.

2. The hinged frame (20) according to claim 1, **characterized in that** the orientation device (41) includes a ball joint around which a cylinder (43) pivots, having at least one tapped bore receiving a screw the head whereof is a tightening wheel (42) of said ball joint.

3. The hinged frame (20) according to claim 2, **characterized in that** the ball joint is a sphere attached to a rod, and the cylinder (43) includes a notch capable of receiving said rod when the image forming device (130) is inclined toward the ground.

4. The hinged frame (20) according to claim 1, **characterized in that** the orientation device (141) includes:
- several parts (36), called olives, each having a mutually complementary convex surface and concave surface,
- an insert (34) of which one portion is threaded and arranged within the tubular element (40; 140), and
- a tightening wheel (35) including an internal thread receiving the threaded portion of said insert so as to adjust the flexibility of said tubular element.

5. The hinged frame (20) according to one of claims 1 to 3, **characterized in that** at least a portion of the hinged arm (30) is made of a non-magnetic material.

6. The hinged frame (20) according to one of claims 1 to 4, **characterized in that** the hinged device (50) includes
- a support (62) arranged at the movable end of said device, said support being intended to support the display screen (150),
- several hinges provided with blocking wheels (51; 52),
- a rod (55) extending substantially vertically and having an abutment at each of its ends,
- a tube (56) disposed around said rod and having a lateral tapped bore, and
- a screw disposed within said bore, the head whereof is a tightening wheel (54) of said rod.

7. The hinged frame (20) according to one of claims 1 to 5, **characterized in that** the base (70) is ballasted and **in that** the displacement means are wheels (80) mounted on said base, the rotation whereof is blockable.

8. The hinged frame (20) according to one of claims 1 to 6, **characterized in that** it further includes an electric storage battery intended to supply electricity to the image forming device (130) and/or the display screen (150).

9. A video system (10) intended to be disposed in an operating room, said video system further comprising
- a hinged frame (20) according to one of the preceding claims,
- the image forming device (130) supported by the hinged arm (30), and
- the display screen (150) attached to the hinged support (50).

10. The video system (10) according to claim 8, **characterized in that** it includes
- communication means, and
- a device for recording images taken by the image forming device (130).
